(19) **Europäisches Patentamt**

**European Patent Office**

**Office européen des brevets**

(11) **EP 0 728 843 B1**

(12) **EUROPÄISCHE PATENTSCHRIFT**

(45) Veröffentlichungstag und Bekanntmachung des Hinweises auf die Patenterteilung:
**23.04.2003 Patentblatt 2003/17**

(51) Int Cl.[7]: **C12Q 1/26**, C12Q 1/28, C12Q 1/54, C07F 9/54, C07C 211/62, C07F 9/72, C07F 9/74, C07C 381/12, C07C 391/00

(21) Anmeldenummer: **96100894.3**

(22) Anmeldetag: **23.01.1996**

(54) **Störungsvermindertes Redox-Nachweissystem**

Interference-free redox detection system

Système de détection redox libre d'interférences

(84) Benannte Vertragsstaaten:
**AT BE CH DE DK ES FR GB IT LI LU NL PT SE**

(30) Priorität: **23.02.1995 DE 19506262**

(43) Veröffentlichungstag der Anmeldung:
**28.08.1996 Patentblatt 1996/35**

(73) Patentinhaber: **Dade Behring Marburg GmbH 35001 Marburg (DE)**

(72) Erfinder:
• **Habenstein, Klaus, Dr.
D-35083 Wetter (DE)**
• **Zopf, Dieter
D-35041 Marburg (DE)**
• **Bursch, Winfried
D-35039 Marburg (DE)**

(56) Entgegenhaltungen:
EP-A- 0 123 115          EP-A- 0 141 244
EP-A- 0 223 221          EP-A- 0 480 340
EP-A- 0 513 594          US-A- 3 904 481

• **TRANSITION MET. CHEM., Bd. 14, Nr. 3, 1989, Seiten 230-232, XP002004995 A. C. DENGEL ET AL.: "Tetraphenylphosphonium perosmate (VII) as an oxidant: comparison of (OsO4)- with perruthenate."**
• **TETRAHEDRON LETTERS, Bd. 24, Nr. 34, 1983, Seiten 3631-3634, XP002004996 T. TAKATA ET AL.: "Mild and selective oxygen atom transfer: Bu4NIO4 with metalloporphyrins."**

EP 0 728 843 B1

**Beschreibung**

Die vorliegende Erfindung betrifft Reagenzien und Verfahren zur Herstellung von Diagnostika, die ein chromogenes Redox-Nachweissystem verwenden und deren chromogenes Redox-Nachweissystem im wesentlichen von Störungen durch reduzierende Verbindungen in dem Probenmaterial unbeeinflußt bleibt.

Solche Redox-Nachweissysteme werden sowohl bei naßchemischen als auch bei sogenannten trockenchemischen Testverfahren eingesetzt. Insbesondere werden dabei Nachweissysteme eingesetzt, in denen der Nachweis mit Hilfe einer Redox-Kette geführt wird. Diese kann bestehen aus einer Oxidase, die mit dem Analyten unter Freisetzung von $H_2O_2$ reagiert, einer Peroxidase als Elektronen-Überträger und einem Redox-Indikator als Elektronendonator. Sie kann aber auch aus einem Analyten mit elektronenübertragenden Eigenschaften (Pseudoperoxidase), einem starken Oxidationsmittel und dem Redox-Indikator als Elektronendonator gebildet werden.

Trockenchemische Testsysteme als solche sind dem Fachmann an sich bekannt.

Diese Nachweissysteme werden systembedingt durch redoxaktive Substanzen, insbesondere reduzierende Verbindungen, gestört.

Es wurden mehrere Verfahren beschrieben, die solch störende Probeninhaltsstoffe vor Ausführung von Analysen mit Hilfe oxidierender Medien aus dem Probenmaterial entfernen:

Als Oxidationsmittel werden dabei z. B. Ascorbatoxidase (EP-A 0 016 962), Schwermetallsalze (US 3,411,887), Übergangsmetalle (EP-A 0 041 188), Eisenkomplexe (EP-A 0 123 115) oder Perjodate (EP-A 0 103 958) eingesetzt.

Die Ascorbatoxidase wirkt sehr träge und ist damit inbesondere für einen "trockenchemischen" Schnelltest wenig geeignet. Die genannten Schwermetalle haben zumeist intensive Eigenfarben, die sich bei Reaktion mit Ascorbinsäure zudem noch deutlich ändern, so daß die eigentliche chromogene Nachweisreaktion schwer erkennbar ist. Die Verwendung von Perjodat-haltigen Reagenzien als starker Sauerstoffdonor (Tetrahydron Letters, Bd. 24, 1983, S. 3631-3634) oder in Nachweisverfahren (EP-A-0 141 244 oder EP-A-0 223 221) ist bekannt. Perjodate lassen sich zwar auf Papiere imprägnieren, gehen dabei jedoch mit der Cellulose eine.quantitative Umsetzung zu Kohlenhydrataldehyden und Jodat ein.

Jodat reagiert mit den üblicherweise verwendeten Chromogenen in einer langsamen Reaktion, die auf Testpapieren zu einer Eigenanfärbung während der Testphase führt (also falsch-positive Farbanzeige). Dies kann man durch aufwendige Überlagerung mehrerer Testpapiere (EP-A 0 037 056) lösen, wodurch Jodat und Indikatorfarbstoff räumlich voneinander getrennt werden.

Die unlöslichen Oxidationsverbindungen gehören im allgemeinen zu den oben erwähnten Schwermetallverbindungen mit starker Eigenfarbe. Zudem oxidieren auch diese Verbindungen die verwendeten Indikatorfarbstoffe mit falsch-positiver Farbanzeige.

Die Eisenkomplexverbindungen (EP-A 0 123 115) benötigen ein weiteres Oxidationsmittel zur Aufoxidation des vom Reduktionsmittel (beispielsweise Ascorbinsäure) reduzierten Eisensalzes, wie in der EP-A - 0 513 594 beschrieben wird.

Obwohl die Lösungen nach dem Stand der Technik auch bereits einen gewissen Vorteil erbrachten, bestand dennoch Bedarf für ein Redox-Nachweissystem, das im wesentlichen unbeeinflußt bleibt durch reduzierende Bestandteile einer Probe.

Der vorliegenden Erfindung lag somit das technische Problem zugrunde, Reagenzien zu finden, die in einem Redox-Nachweissystem die Störungen durch reduzierende Substanzen verhindern und gleichzeitig die verwendeten chromogenen, bzw. die daraus gebildeten farbigen Stoffe unbeeinflußt lassen, bzw. sogar stabilisieren.

Die Lösung dieses technischen Problems erfolgt durch die Bereitstellung der in den Patenansprüchen beschriebenen Ausführungsformen.

Es wurde überraschenderweise festgestellt, daß die Lösung durch eine Komplexierung des als extrem oxidativ wirksam bekannten Perjodat-Ions, gefunden wurde, wobei als geeignete Komplexbildner für das Perjodat-Ion Substanzen der folgenden Formel **I**

$$[Z(R_n)]^{(+)} A^{(-)} \qquad \textbf{(I)}$$

worin

Z     = P, N, As, S, Se
n     = 4, wenn Z = P, N, As und 3, wenn Z = S und Se
R     = Phenyl, Benzyl, Alkyl, Cycloalkyl, die jeweils auch mit C1-8 Alkyl substituiert sein können und
$A^{(-)}$     =$Br^{(-)}$, $J^{(-)}$, $Cl^{(-)}$, $F^{(-)}$ bedeutet,

bevorzugterweise

Z     = P, N, As und
R     = Phenyl, Benzyl, Cycloalkyl, die jeweils auch mit C1-8 Alkyl substituiert sein können und Alkyl bedeutet,

besonders bevorzugterweise

Z     = P, N, As und
R     = Phenyl, Benzyl, Cycloalkyl, Alkyl, wobei die Summe der C-Atome größer 12 sein soll, wenn R nur aus Alkylresten besteht, bedeutet,

ganz bevorzugterweise

R     = Phenyl, Benzyl, Alkyl, Cycloalkyl, die jeweils auch mit C1-4 Alkyl substituiert sein können, bedeutet.

Diese setzen sich mit Perjodat um zu dem Komplex der Formel **II**

$$R_n Z \, JO_4 \text{ für S, Se} \qquad \textbf{(II)}$$

worin R, n, und Z die oben angeführte Bedeutung haben.

Die hier beschriebenen Komplexe zeigen eine verminderte Löslichkeit in Wasser, lösen sich jedoch gut in bestimmten organischen Lösungsmitteln.

Die erfindungsgemäße Stabilisierung des Oxidationspotentials zeigt sich daran, daß die Komplexe im Gegensatz zum freien Perjodat weder mit der Cellulosefaser des Papiers (bei der Imprägnierung) noch mit Oxidationsindikatoren bei Anwesenheit der üblicherweise bei der Herstellung von Testpapieren verwendeten Stabilisatoren reagieren.

Eine bevorzugte Ausführungsform des erfindungsgemäßen Testpapiers wird auf folgende Weise hergestellt:

a) 1 - 10 g einer mehrbasischen Carbonsäure mit einem pKa von etwa 4 bis 6 - besonders bevorzugt sind dabei Zitronensäure, Tricarballylsäure, Phthalsäure, Maleinsäure und Glutarsäure - werden in 50 ml $H_2O$ gelöst und der pH auf 4.5 -5.5, bevorzugterweise auf etwa 5 eingestellt.

b) 0,1 - 1 g einer gel- undloder filmbildenden Substanz, wie z. B. Gelatine, Polygeline, Mowiol®, Mowilith®, Gantrez®, Celluloseester- und/oder-ether und Povima,
10 - 200 mg eines Komplexbildners, wie z. B. EDTA,
10 - 200 mg eines Untergrundfarbstoffes, wie z. B. Tartrazin und
1 - 10 mg eines Enzymhemmers und Antioxidans, wie z. B. Hydrochinon, Zinnchlorid, Aminonaphtolsulfonsäure oder Ascorbinsäure, werden in 20 ml $H_2O$ gelöst und mit dem unter a) hergestellten Puffer auf 60 ml aufgefüllt.

In 7 ml des unter a) hergestellten Puffers werden je 50 - 200 mg Glucoseoxidase und Peroxidase, vorteilhafterweise in einem Gewichtsverhältnis von etwa 1:1, gelöst und zu der unter b) hergestellten Lösung (60 ml) hinzugefügt. Mit dieser Lösung werden perjodathaltige Papiere, wie z. B. die in Beispiel 2 hergestellten, imprägniert und bei einer geeigneten Temperatur getrocknet. Die so erhaltenen Testeelemente - die vorteilhafterweise auch aus synthetischen Trägern, wie z. B. Membranen, wobei zellulosehaltige Membranen bevorzugt sind, die nach dem erfindungsgemäßen Verfahren mit perjodathaltiger Lösung imprägniert werden, bestehen können - werden anschließend mit einer Lösung von 10 - 100 mg eines Peroxidasesubstrates, wie z. B. Tetramethylbenzidin in 10 ml eines organischen Lösungsmittels, wie z. B. Toluol, getränkt. Das so erhaltene Testelement wird auf geeignete Weise getrocknet.

Die folgenden Beispiele erläutern die Erfindung.

Wie in den Beispielen gezeigt wird, lassen sich die oben genannten Verbindungen ohne Zersetzung auf Papier imprägnieren, was mit freiem Perjodat nicht möglich ist.

**Beispiel 1:**

**Herstellung von Benzyltriphenylphosphoniumperjodat (BTPP)**

Lösung 1:      5 g Natriumperjodat in 250 ml E-Wasser
Lösung 2: ,     5 g Benzyltriphenylphosphoniumchlorid in 500 ml E-Wasser

Lösung 2 wird innerhalb 10 min unter Rühren in Lösung 1 getropft. Es wird weitere 5 min nachgerührt und anschließend 10 min stehen gelassen. Der Niederschlag wird abfiltriert, in 200 ml E-Wasser resuspendiert und nach erneuter Filtration getrocknet.

**Ausbeute:** 6,6 g Benzyltriphenylphosphoniumperjodat

In analoger Weise lassen sich auch die folgenden Komplexverbindungen herstellen:

Cyclohexyltriphenylphosphoniumperjodat, Benzethoniumperjodat, Hexadecyltrimethylammoniumperjodat, Dodecylpyridiniumperjodat, Tetradodecylammoniumperjodat, Tetraphenylarsoniumperjodat und Ethoxycarbonylmethyl-dimethylsulfoniumperjodat.

Bei den stärker hydrophoben Verbindungen dieser Serie wird Lösung 2 vorteilhafterweise mit einer Äthanol/Wasser-Mischung angesetzt

**Beispiel 2:**

**Imprägnierung von Benzyltriphenylphosphoniumperjodat (BTPP) auf Papier:**

**A) Imprägnierung mit organisch/wäßriger Lösung:**

Indikatorrohpapier SS2316 der Firma Schleicher und Schüll wird etwa 2 min in eine Tränkschale mit einer 1 %igen Lösung von BTPP in Aceton/Wasser (6 + 4) getaucht, der Überschuß an Tränklösung wird zwischen zwei Glasstäben abgestreift und das Papier wird 5 min bei 80 °C im Umlufttrockenschrank getrocknet.

Auf dem so hergestellten Papier lassen sich, nach dem Waschen mit E-Wasser, folgende Verbindungen nachweisen:

$$JO_4^-: 58\ \mu g/cm^2$$

**B) Direktfällung:**

Lösung 1:10 g Benzyltriphenylphosphoniumchlorid werden in 1000 ml E-Wasser gelöst.

Lösung 2: 5 g Natriumperjodat werden in 1000 ml E-Wasser gelöst.

Indikatorrohpapier T86 der Firma J. C. Binzer wird nacheinander mit Lösung 1 und Lösung 2 wie unter A beschrieben, behandelt. Auf dem so hergestellten Papier lassen sich, nach dem Waschen mit E-Wasser, folgende Verbindungen nachweisen:

$$JO_4^-: 44\ \mu g/cm^2$$

Wird die Reihenfolge der Tränklösungen vertauscht, so erfolgt bei der Imprägnierung der Perjodatlösung die bereits zuvor beschriebene, weitgehende Umsetzung des Perjodats mit der Cellulose unter Bildung von Jodat. Die verbleibende Perjodatmenge reagiert mit dem Phosphoniumchlorid unter Bildung des sehr schwer löslichen BTPP und kann folglich nach dem Waschen noch auf dem Papier nachgewiesen werden:

$$JO_4^-: 10\ \mu g/cm^2$$

Das Jodat ist im Gegensatz zum BTPP gut wasserlöslich und läßt sich durch Nachwaschen leicht vom Papier entfernen, wie folgender Versuch zeigt:

Lösung 1: 10 g Benzyltriphenylphosphoniumchlorid werden in 1000 ml E-Wasser gelöst.

Lösung 2: 5 g Natriumjodat werden in 1000 ml E-Wasser gelöst.

Indikatorrohpapier T86 der Firma J. C. Binzer wird nacheinander mit Lösung 1 und Lösung 2 wie unter B beschrieben, behandelt. Auf dem so hergestellten Papier läßt sich, nach dem Waschen mit E-Wasser, die Verbindung $JO_3^-$ nicht mehr nachweisen.

Ein analoges Verhalten zeigen auch die übrigen im Beispiel 1 genannten Verbindungen.

**Beispiel 3:**

**Herstellung eines Testpapiers für den Nachweis von Glucose in Probeflüssigkeiten**

In 50 ml E-Wasser werden 7 g Natriumcitrat gelöst und der pH-Wert wird auf pH 5 eingestellt. In 20 ml E-Wasser werden 0,3 g Gelatine, 70 mg EDTA, 50 mg Tartrazin und 2 mg Hydrochinon gelöst. Danach wird diese Lösung mit dem Citratpuffer auf 60 ml aufgefüllt. In 7 ml des Citratpuffers werden je 100 mg Glucoseoxidase und Peroxidase gelöst und zu den obigen 60 ml hinzugefügt. Mit dieser Reagenzienlösung werden perjodathaltige Papiere (hergestellt nach Beispiel 2) imprägniert und im Umlufttrockenschrank bei 50 °C getrocknet. Danach werden die Papiere in eine Lösung von 30 mg Tetramethylbenzidin in 10 ml Toluol getaucht und das imprägnierte Papier wird wiederum bei 50 °C im Umlufttrockenschrank getrocknet.

Das so erhaltene Glucose-Testpapier ergibt beim Eintauchen in eine Probeflüssigkeit mit einem Gehalt von 1 g Glucose und 2 g Ascorbinsäure pro Liter eine grüne Farbanzeige. Im Vergleich wird mit einem Testpapier ohne die erfindungsgemäßen Perjodatkomplexe keine Farbanzeige erhalten.

**Patentansprüche**

1. Verwendung eines Reagenzes enthaltend einen Komplexbildner der allgemeinen Formel I

$$[Z(R_n)]^{(+)}\ A^{(-)} \qquad (I)$$

worin

Z = P, N, As, S, Se
n = 4, wenn Z = P, N, As and 3, wenn Z = S and Se
R = Phenyl, Benzyl, Alkyl, Cycloalkyl, die jeweils auch mit C1-8 Alkyl substituiert sein können, und wobei R gleich oder ungleich sein kann, und A(-) = Br (-), J(-), Cl(-), F(-) bedeutet;

und Perjodat-Ionen in einem trockenchemischen Nachweisverfahren; bevorzugt in einem trockenchemischen Verfahren auf der Basis von Indikatorpapieren.

2. Verwendung nach Anspruch 1, worin

Z = P, N, As, und
R = Phenyl, Benzyl, Cycloalkyl, die jeweils auch mit C1-8 Alkyl substituiert sein können, und Alkyl, und wobei R gleich oder ungleich sein kann, bedeutet.

3. Verwendung nach Anspruch 1, worin

Z = P, N, As, und
R = Phenyl, Benzyl, Cycloalkyl, Alkyl, wobei die Summe der C-Atome größer 12 sein soll, wenn R nur aus Alkylresten besteht, und wobei R gleich oder ungleich sein kann, bedeutet.

4. Verwendung nach Anspruch 1, worin

R = Phenyl, Benzyl, Alkyl, Cycloalkyl, die jeweils auch mit C1-4 Alkyl substituiert sein können, und wobei R gleich oder ungleich sein kann, bedeutet.

5. Verwendung nach einem der Ansprüche 1-4 zur Bestimmung eines Analyten in einer Probe einer biologischen Flüssigkeit.

6. Verwendung nach Anspruch 5, wobei der Analyt ausgewählt ist aus der folgenden Gruppe: Glucose, Harnsäure, Peroxidase, Pseudoperoxidase.

7. Trockenchemisches Element zum Nachweis und zur Bestimmung eines Analyten in einer Probe einer biologischen Flüssigkeit, **dadurch gekennzeichnet, daß** es eine Komplexverbindung der allgemeinen Formel II enthält,

$$R_n Z\, JO_4 \qquad\qquad (II)$$

worin

Z     = P, N, As, S, Se
n =     4, wenn Z = P, N, As und 3, wenn Z = S und Se, und
R =     Phenyl, Benzyl, Alkyl, Cycloalkyl, die jeweils auch mit C1-8 Alkyl substituiert sein können, und wobei R gleich oder ungleich sein kann, bedeutet

8. Trockenchemisches Element gemäß Anspruch 7, worin

Z =     P, N, As, und
R =     Phenyl, Benzyl, Cycloalkyl, die jeweils auch mit C1-8 Alkyl substituiert sein können, und Alkyl, und wobei R gleich oder ungleich sein kann, bedeutet.

9. Trockenchemisches Element gemäß Anspruch 7, worin

Z =     P, N, As, und
R =     Phenyl, Benzyl, Cycloalkyl, Alkyl, wobei die Summe der C-Atome größer 12 sein soll, wenn R nur aus Alkylresten besteht, und wobei R gleich oder ungleich sein kann, bedeutet.

10. Trockenchemisches Element gemäß Anspruch 7, worin

R =     Phenyl, Benzyl, Alkyl, Cycloalkyl, die jeweils auch mit C1-4 Alkyl substituiert sein können, und wobei R gleich oder ungleich sein kann, bedeutet

11. Trockenchemisches Element gemäß Anspruch 7, welches die Komplexverbindung Benzyltriphenylphosphoniumperjodat, Cyclohexyltriphenylphosphoniumperjodat, Benzethoniumperjodat, Hexadecyltrimethylammoniumperjodat, Dodecylpyridiniumperjodat, Tetradodecylammoniumperjodat, Tetraphenylarsoniumperjodat oder Ethoxycarbonylmethyl-dimethylsulfoniumperjodat enthält.

12. Verfahren zur Herstellung eines trockenchemischen Testelements, **dadurch gekennzeichnet, daß** Papier mit einer Komplexverbindung der allgemeinen Formel II imprägniert wird,

$$R_n Z\, JO_4 \qquad\qquad (II)$$

worin

Z     = P, N, As, S, Se
n =     4, wenn Z = P, N, As und 3, wenn Z = S und Se, und
R =     Phenyl, Benzyl, Alkyl, Cycloalkyl, die jeweils auch mit C1-8 Alkyl substituiert sein können, und wobei R gleich oder ungleich sein kann, bedeutet.

13. Herstellungsverfahren nach Anspruch 12, worin

Z =     P, N, As, und
R =     Phenyl, Benzyl, Cycloalkyl, die jeweils auch mit C1-8 Alkyl substituiert sein können, und Alkyl, und wobei R gleich oder ungleich sein kann, bedeutet.

14. Herstellungsverfahren nach Anspruch 12, worin

Z =     P, N, As, und
R =     Phenyl, Benzyl, Cycloalkyl, Alkyl, wobei die Summe der C-Atome größer 12 sein soll, wenn R nur aus Alkylresten besteht, und wobei R gleich oder ungleich sein kann, bedeutet.

15. Herstellungsverfahren nach Anspruch 12, worin

R = Phenyl, Benzyl, Alkyl, Cycloalkyl, die jeweils auch mit C1-4 Alkyl substituiert sein können, und wobei R gleich oder ungleich sein kann, bedeutet.

16. Herstellungsverfahren nach Anspruch 12, **dadurch gekennzeichnet, daß** Papier mit Benzyltriphenylphosphoniumperjodat, Cyclohexyltriphenylphosphoniumperjodat, Benzethoniumperjodat, Hexadecyltrimethylammoniumperjodat, Dodecylpyridiniumperjodat, Tetradodecylammoniumperjodat, Tetraphenylarsoniumperjodat oder Ethoxycarbonylmethyl-dimethylsulfoniumperjodat imprägniert wird,

17. Diagnostisches Verfahren zum Nachweis und zur Bestimmung eines Analyten in einer Probe einer biologischen Flüssigkeit, **dadurch gekennzeichnet, daß** ein trockenchemisches Element nach einem der Ansprüche 7-11 verwendet wird.

18. Diagnostisches, ein Redox-Nachweisreaktion verwendendes Verfahren zum Nachweis und zur Bestimmung eines Analyten in einer Probe einer biologischen Flüssigkeit **dadurch gekennzeichnet, daß** ein Komplexbildener der allgemeinen Formel I

$$[Z(R_n)]^{(+)} A^{(-)} \qquad (I)$$

worin

Z = P, N, As, S, Se
n = 4, wenn Z = P, N, As und 3, wenn Z = S und Se
R = Phenyl, Benzyl, Alkyl, Cycloalkyl, die jeweils auch mit C1-8 Alkyl substituiert sein können, und wobei R gleich oder ungleich sein kann, und

A(-) = Br (-), J(-), Cl(-), F(-) bedeutet,
zur Komplexierung von Perjodat-Ionen verwendet wird und die Redox-Nachweisreaktion in Gegenwart des Komplexbildners und Perjodat-Ionen stattfindet.

19. Diagnostisches, ein Redox-Nachweisreaktion verwendendes Verfahren zum Nachweis und zur Bestimmung eines Analyten in einer Probe einer biologischen Flüssigkeit, **dadurch gekennzeichnet, daß** die Redox-Nachweisreaktion in Gegenwart von Benzyltriphenylphosphoniumperjodat, Cyclohexyltriphenylphosphoniumperjodat, Benzethoniumperjodat, Hexadecyltrimethylammoniumperjodat, Dodecylpyridiniumperjodat, Tetradodecylammoniumperjodat, Tetraphenylarsoniumperjodat oder Ethoxycarbonylmethyl-dimethylsulfoniumperjodat stattfindet.

**Claims**

1. The use of a reagent comprising a complexing agent of the formula I

$$[Z(R_n)]^{(+)} A^{(-)} \qquad (I)$$

in which

Z is P, N, As, S or Se,
n is 4 if Z = P, N and As and 3 if Z = S and Se
R is phenyl, benzyl, alkyl or cycloalkyl, each of which may also be substituted by C1-8 alkyl, and where R may be identical or different, and

$A^{(-)}$ is $Br^{(-)}$, $I^{(-)}$, $Cl^{(-)}$ or $F^{(-)}$;
and periodate ions in a dry-chemical detection process; preferably in a dry-chemical process based on indicator papers.

2. The use as claimed in claim 1, in which

Z is P, N or As, and
R is phenyl, benzyl, cycloalkyl, each of which may also be substituted by C1-8 alkyl, and alkyl, and where R may be identical or different.

3. The use as claimed in claim 1, in which

Z is P, N or As, and
R is phenyl, benzyl, cycloalkyl or alkyl, where the sum of the C atoms is to be greater than 12 if R consists only of alkyl radicals, and where R may be identical or different.

4. The use as claimed in claim 1, in which

R is phenyl, benzyl, alkyl or cycloalkyl, each of which may also be substituted by C1-4 alkyl, and where R may be identical or different.

5. The use as claimed in any of claims 1-4 for the determination of an analyte in a sample of biological fluid.

**6.** The use as claimed in claim 5, the analyte being selected from the following group: glucose, uric acid, peroxidase, pseudoperoxidase.

**7.** A dry-chemical element for the detection and determination of an analyte in a sample of a biological fluid, which element comprises a complex compound of the formula II

$$R_nZIO_4 \qquad (II)$$

in which

Z is    P, N, As, S or Se,
n is    4 if Z = P, N and As and 3 if Z = S and Se, and
R is    phenyl, benzyl, alkyl or cycloalkyl, each of which may also be substituted by C1-8 alkyl, and where R may be identical or different.

**8.** A dry-chemical element as claimed in claim 7, in which

Z is    P, N or As, and
R is    phenyl, benzyl or cycloalkyl, each of which may also be substituted by C1-8 alkyl, and alkyl, and where R may be identical or different.

**9.** A dry-chemical element as claimed in claim 7, in which

Z is    P, N or As, and
R is    phenyl, benzyl, cycloalkyl or alkyl, where the sum of the C atoms is to be greater than 12 if R consists only of alkyl radicals, and where. R may be identical or different.

**10.** A dry-chemical element as claimed in claim 7, in which

R is    phenyl, benzyl, alkyl or cycloalkyl, each of which may also be substituted by C1-4 alkyl, and where R may be identical or different.

**11.** A dry-chemical element as claimed in claim 7, which comprises the complex compound benzyltriphenylphosphonium periodate, cyclohexyltriphenylphosphonium periodate, benzethonium periodate, hexadecyltrimethylammonium periodate, dodecylpyridinium periodate, tetradodecylammonium periodate, tetraphenylarsonium periodate or ethoxycarbonylmethyldimethylsulfonium periodate.

**12.** A process for preparing a dry-chemical test element, which comprises impregnating paper with a complex compound of the formula II

$$R_nZIO_4 \qquad (II)$$

in which

Z is    P, N, As, S or Se,
n is    4 if Z = P, N and As and 3 if Z = S and Se, and
R is    phenyl, benzyl, alkyl or cycloalkyl, each of which may also be substituted by C1-8 alkyl, and where R may be identical or different.

**13.** The preparation process as claimed in claim 12, in which

Z is    P, N or As, and
R is    phenyl, benzyl or cycloalkyl, each of which may also be substituted by C1-8 alkyl, and alkyl, and where R may be identical or different.

**14.** The preparation process as claimed in claim 12, in which

Z is    P, N or As, and
R is    phenyl, benzyl, cycloalkyl or alkyl, where , the sum of the C atoms is to be greater than 12 if R consists only of alkyl radicals, and where R may be identical or different.

**15.** The preparation process as claimed in claim 12, in which

R is    phenyl, benzyl, alkyl or cycloalkyl, each of which may also be substituted by C1-4 alkyl, and where R may be identical or different.

**16.** The preparation process as claimed in claim 12, wherein paper is impregnated with benzyltriphenylphosphonium periodate, cyclohexyltriphenylphosphonium periodate, benzethonium periodate, hexadecyltrimethylammonium periodate, dodecylpyridinium periodate, tetradodecylammonium periodate, tetraphenylarsonium periodate or ethoxycarbonylmethyldimethylsulfonium periodate.

**17.** A diagnostic process for the detection and determination of an analyte in a sample of a biological fluid, which comprises using a dry-chemical element as claimed in any of claims 7-11.

**18.** A diagnostic method using a redox detection reaction for the detection and determination of an analyte in a sample of a biological fluid, which comprises using a complexing agent of the formula I

$$[Z(R_n)]^{(+)} A^{(-)} \qquad (I)$$

in which

Z is P, N, As, S or Se,

n is 4 if Z = P, N and As and 3 if Z = S and Se

R is phenyl, benzyl, alkyl or cycloalkyl, each of which may also be substituted by C1-8 alkyl, and where R may be identical or different, and

$A^{(-)}$ is $Br^{(-)}$, $I^{(-)}$, $Cl^{(-)}$ or $F^{(-)}$,

for complexing periodate ions, and the redox detection reaction takes place in the presence of complexing agent and periodate ions.

19. A diagnostic process using a redox detection reaction for the detection and determination of an analyte in a sample of a biological fluid, which comprises the redox detection reaction taking place in the presence of benzyltriphenylphosphonium periodate, cyclohexyltriphenylphosphonium periodate, benzethonium periodate, hexadecyltrimethylammonium periodate, dodecylpyridinium periodate, tetradodecylammonium periodate, tetraphenylarsonium periodate or ethoxycarbonylmethyldimethylsulfonium periodate.

## Revendications

1. Utilisation d'un réactif contenant un complexant de formule générale I

$$[Z(R_n)]^{(+)} A^{(-)} \qquad (I)$$

dans laquelle

Z représente P, N, As, S, Se

n = 4 lorsque Z = P, N, As, et 3 lorsque Z = S et Se

R représente un groupe phényle, benzyle, alkyle, cycloalkyle, qui peuvent également être substitués chacun par un groupe alkyle en $C_{1-8}$, et les radicaux R pouvant être identiques ou différents, et

$A^{(-)}$ représente $Br^{(-)}$, $I^{(-)}$, $Cl^{(-)}$, $F^{(-)}$ ;
et d'ions periodate, dans un procédé de détection par voie chimique sèche ; de préférence dans un procédé de détection par voie chimique sèche à base de papiers indicateurs.

2. Utilisation selon la revendication 1, dans laquelle

Z représente P, N, As et

R représente un groupe phényle, benzyle, cycloalkyle, qui peuvent également être substitués chacun par un groupe alkyle en $C_{1-8}$, et

alkyle, et les radicaux R pouvant être identiques ou différents.

3. Utilisation selon la revendication 1, dans laquelle

Z représente P, N, As et

R représente un groupe phényle, benzyle, cycloalkyle, alkyle, la somme des atomes de carbone devant être supérieure à 12 lorsque R n'est constitué que de radicaux alkyle et les radicaux R pouvant être identiques ou différents.

4. Utilisation selon la revendication 1, dans laquelle

R représente un groupe phényle, benzyle, alkyle, cycloalkyle, qui peuvent également être substitués chacun par un groupe alkyle en $C_{1-4}$, et les radicaux R pouvant être identiques ou différents.

5. Utilisation selon l'une quelconque des revendications 1 à 4, pour la détermination d'un analyte dans un échantillon d'un liquide biologique.

6. Utilisation selon la revendication 5, dans laquelle l'analyte est choisi dans le groupe suivant : le glucose, l'acide urique, la peroxydase, la pseudoperoxydase.

7. Elément utilisé par voie chimique sèche, pour la détection et la détermination d'un analyte dans un échantillon d'un liquide biologique, **caractérisé en ce qu'**il contient un composé complexe de formule générale II

$$R_n ZIO_4 \qquad (II)$$

dans laquelle

Z représente P, M, As, S, Se

n = 4 lorsque Z = P, N, As, et 3 lorsque Z = S et Se, et

R représente un groupe phényle, benzyle, alkyle, cycloalkyle, qui peuvent également être substitués chacun par un groupe alkyle en $C_{1-8}$, et les radicaux R pouvant être identiques ou différents.

8. Elément utilisé par voie chimique sèche selon la revendication 7, dans lequel

Z représente P, N, As et

R représente un groupe phényle, benzyle, cycloalkyle, qui peuvent également être substitués chacun par un groupe alkyle en $C_{1-8}$, et

alkyle, et les radicaux R pouvant être identiques ou différents.

**9.** Elément utilisé par voie chimique sèche selon la revendication 7, dans lequel

Z représente P, N, As et

R représente un groupe phényle, benzyle, cycloalkyle, alkyle, la somme des atomes de carbone devant être supérieure à 12 lorsque R n'est constitué que de radicaux alkyle et les radicaux R pouvant être identiques ou différents.

**10.** Elément utilisé par voie chimique sèche selon la revendication 7, dans lequel R représente un groupe phényle, benzyle, alkyle, cycloalkyle, qui peuvent également être substitués chacun par un groupe alkyle en $C_{1-4}$, et les radicaux R pouvant être identiques ou différents.

**11.** Elément utilisé par voie chimique sèche selon la revendication 7, contenant le composé complexe periodate de benzyltriphénylphosphonium, du periodate de cyclohexyltriphénylphosphonium, du periodate de benzéthonium, du periodate d'hexadécyltriméthylammonium, du periodate de dodécylpyridinium, du periodate de tétradodécylammonium, du periodate de tétraphénylarsonium ou du periodate d'éthoxycarbonylméthyl-diméthylsulfonium.

**12.** Procédé pour la fabrication d'un élément de test par voie chimique sèche, **caractérisé en ce qu'**on imprègne du papier avec un composé complexe de formule générale II

$$R_nZIO_4 \qquad (II)$$

dans laquelle

Z représente P, N, As, S, Se
n = 4 lorsque Z = P, N, As, et 3 lorsque Z = S et Se, et
R représente un groupe phényle, benzyle, alkyle, cycloalkyle, qui peuvent également être substitués chacun par un groupe alkyle en $C_{1-8}$, et les radicaux R pouvant être identiques ou différents.

**13.** Procédé de fabrication selon la revendication 12, dans lequel

Z représente P, N, As et
R représente un groupe phényle, benzyle, cycloalkyle, qui peuvent également être substitués chacun par un groupe alkyle en $C_{1-8}$, et alkyle, et les radicaux R pouvant être identiques ou différents.

**14.** Procédé de fabrication selon la revendication 12, dans lequel

Z représente P, N, As et

R représente un groupe phényle, benzyle, cycloalkyle, alkyle, la somme des atomes de carbone devant être supérieure à 12 lorsque R n'est constitué que de radicaux alkyle et les radicaux R pouvant être identiques ou différents.

**15.** Procédé de fabrication selon la revendication 12, dans lequel

R représente un groupe phényle, benzyle, alkyle, cycloalkyle, qui peuvent également être substitués chacun par un groupe alkyle en $C_{1-4}$, et les radicaux R pouvant être identiques ou différents.

**16.** Procédé de fabrication selon la revendication 12, **caractérisé en ce que** le papier est imprégné avec du periodate de benzyltriphénylphosphonium, du periodate de cyclohexyltriphénylphosphonium, du periodate de benzéthonium, du periodate d'hexadécyltriméthylammonium, du periodate de dodécylpyridinium, du periodate de tétradodécylammonium, du periodate de tétraphénylarsonium ou du periodate d'éthoxycarbonylméthyl-diméthylsulfonium.

**17.** Procédé de diagnostic pour la détection et pour la détermination d'un analyte dans un échantillon d'un liquide biologique, **caractérisé en ce qu'**on utilise un élément de test par voie chimique sèche selon l'une quelconque des revendications 7 à 11.

**18.** Procédé de diagnostic utilisant une réaction de détection rédox, pour la détection et pour la détermination d'un analyte dans un échantillon d'un liquide biologique, **caractérisé en ce qu'**on utilise un complexant de formule générale I

$$[Z(R_n)]^{(+)} A^{(-)} \qquad (I)$$

dans laquelle

Z représente P, N, As, S, Se
n = 4 lorsque Z = P, N, As, et 3 lorsque Z = S et Se
R représente un groupe phényle, benzyle, alkyle, cycloalkyle, qui peuvent également être substitués chacun par un groupe alkyle en $C_{1-8}$, et les radicaux R pouvant être identiques ou différents, et

$A^{(-)}$ représente $Br^{(-)}$, $I^{(-)}$, $Cl^{(-)}$, $F^{(-)}$, pour complexer des ions periodate et la réaction de détection rédox a lieu en présence du complexant et d'ions periodate.

**19.** Procédé de diagnostic utilisant une réaction de détection rédox, pour la détection et la détermination d'un analyte dans un échantillon d'un liquide biologique, **caractérisé en ce que** la réaction de détection rédox a lieu en présence de periodate de benzyltriphénylphosphonium, periodate de cyclohexyltriphénylphosphonium, periodate de benzéthonium, periodate d'hexadécyltriméthylammonium, periodate de dodécylpyridinium, periodate de tétradodécylammonium, periodate de tétraphénylarsonium ou periodate d'éthoxycarbonylméthyl-diméthylsulfonium.